Europäisches Patentamt
European Patent Office
Office européen des brevets

(19) (11) Veröffentlichungsnummer: **0 168 608**
**A2**

# (12) EUROPÄISCHE PATENTANMELDUNG

(21) Anmeldenummer: **85106804.9**

(22) Anmeldetag: **03.06.85**

(51) Int. Cl.⁴: **C 07 D 239/34**, C 07 D 239/38, A 01 N 43/54

(30) Priorität: **14.06.84 DE 3422077**

(43) Veröffentlichungstag der Anmeldung: **22.01.86**
**Patentblatt 86/4**

(84) Benannte Vertragsstaaten: **AT BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Sasse, Klaus, Dr., Pützweg 13,**
**D-5060 Bergisch-Gladbach 2 (DE)**
Erfinder: **Eue, Ludwig, Dr., Paul-Klee-Strasse 36,**
**D-5090 Leverkusen 1 (DE)**
Erfinder: **Santel, Hans-Joachim, Gerstenkamp 19,**
**D-5000 Köln 80 (DE)**
Erfinder: **Schmidt, Robert, R. Dr., Im Waldwinkel 110,**
**D-5060 Bergisch-Gladbach 2 (DE)**

## (54) Substituierte Carbonsäureanilide.

(57) Neue substituierte Carbonsäureanilide der Formel

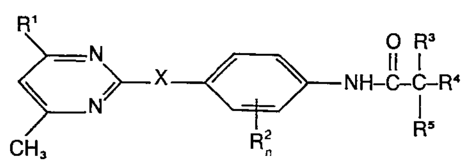

in welcher

X für Sauerstoff oder Schwefel steht,

$R^1$ für Wasserstoff, Halogen, Alkyl mit 1 bis 6 Kohlenstoffatomen, Trifluormethyl, gegebenenfalls substituiertes Phenyl, Alkoxy mit 1 bis 6 Kohlenstoffatomen oder Alkylmercapto mit 1 bis 6 Kohlenstoffatomen steht,

$R^2$ für Halogen oder Methyl steht,

n für 0, 1 oder 2 steht,

$R^3$ für Wasserstoff, Halogen, gegebenenfalls substituiertes Alkyl mit 1 bis 6 Kohlenstoffatomen, gegebenenfalls substituiertes Aryl, gegebenenfalls substituiertes Aralkyl oder für die Reste $-OR^6$ oder $-SOm-R^6$ steht, wobei

$R^6$ für gegebenenfalls substituiertes Alkyl mit 1 bis 6 Kohlenstoffatomen oder für gegebenenfalls substituiertes Aryl steht und

m für 0, 1 oder 2 steht, und

$R^4$ und $R^5$ unabhängig voneinander für Halogen oder gegebenenfalls substituiertes Alkyl mit 1 bis 6 Kohlenstoffatomen stehen oder

$R^4$ und $R^5$ gemeinsam mit dem angrenzenden Kohlenstoffatom für einen gegebenenfalls substituierten, gesättigten oder ungesättigten carbocyclischen Ring mit 3 bis 8 Ring-Kohlenstoffatomen stehen,

mehrere Verfahren zur Herstellung der neuen Stoffe und deren Verwendung als Herbizide.

EP 0 168 608 A2

BAYER AKTIENGESELLSCHAFT 5090 Leverkusen, Bayerwerk
Konzernverwaltung RP
Patentabteilung Dü/Ja-c
Ib

Substituierte Carbonsäureanilide

Die vorliegende Erfindung betrifft neue substituierte Carbonsäureanilide, mehrere Verfahren zu ihrer Herstellung und ihre Verwendung als Herbizide.

Es ist bereits bekannt, daß bestimmte Carbonsäureanilide herbizide Eigenschaften besitzen (vgl. R. Wegler "Chemie der Pflanzenschutz- und Schädlingsbekämpfungsmittel" Bd. 2, Seiten 311-314, Springer-Verlag, Berlin 1970). So kann zum Beispiel das Propionsäure-3,4-dichloranilid zur Unkrautbekämpfung eingesetzt werden. Die Wirkung dieser Verbindung ist gut, jedoch werden bei geringen Aufwandmengen einige Unkräuter nicht immer voll erfaßt. Außerdem läßt auch die Selektivität in manchen Fällen zu wünschen übrig.

Ferner ist bekannt, daß zahlreiche Pyrimidinyl-2-ether und -thioether als Herbizide geeignet sind (vgl. JP-OS 9 474/ 1967, US-PS 3, 126, 271 und US-PS 3, 250, 775). Zum Beispiel können das 2-Phenoxy-4,6-dimethyl-pyrimidin und das 2- (4-Chlor-benzylmer-

capto)-4,6-dimethyl-pyrimidin zur Bekämpfung von Unkräutern verwendet werden. Die herbizide Potenz dieser Stoffe ist aber nicht immer ausreichend.

Weiterhin ist bekannt, daß niedere Acylderivate von 4-Pyridyloxy-(bzw. thio)-anilinen herbizide Eigenschaften aufweisen (vgl. DE-OS 2, 501, 648, JP-OS 55-122 763 und JP-OS 56-123 970). Darüberhinaus sind auch herbizid wirksame Acylderivate von 4-Pyrimidyloxy-anilinen bekannt, die in der 5-Stellung des Pyrimidylrestes durch Halogen oder Trifluormethyl substituiert sind, dagegen in den Positionen 4 und 6 keinen Substituenten enthalten (vgl. JP-OS 56-029 576). Auch die Wirksamkeit dieser Stoffe ist jedoch für praktische Zwecke nicht immer befriedigend.

Es wurden nun neue substituierte Carbonsäureanilide der Formel

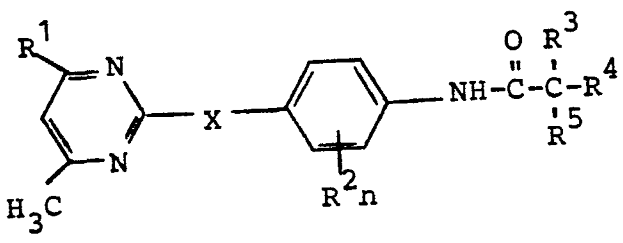

(I)

in welcher

X für Sauerstoff oder Schwefel steht,

$R^1$ für Wasserstoff, Halogen, Alkyl mit 1 bis 6 Kohlenstoffatomen, Trifluormethyl, gegebenenfalls substituiertes Phenyl, Alkoxy mit 1 bis 6 Kohlenstoffatomen oder Alkylmercapto mit 1 bis 6 Kohlenstoffatomen steht,

$R^2$ für Halogen oder Methyl steht,

n für 0,1 oder 2 steht,

$R^3$ für Wasserstoff, Halogen, gegebenenfalls substituiertes Alkyl mit 1 bis 6 Kohlenstoffatomen, gegebenenfalls substituiertes Aryl, gegebenenfalls substituiertes Aralkyl oder für die Reste $-OR^6$ oder $-SO_m-R^6$ steht, wobei

$R^6$ für gegebenenfalls substituiertes Alkyl mit 1 bis 6 Kohlenstoffatomen oder für gegebenenfalls substituiertes Aryl steht und

m für 0,1 oder 2 steht,

und

$R^4$ und $R^5$ unabhängig voneinander für Halogen oder gegebenenfalls substituiertes Alkyl mit 1 bis 6 Kohlenstoffatomen stehen oder

$R^4$ und $R^5$ gemeinsam mit dem angrenzenden Kohlenstoffatom für einen gegebenenfalls substituierten, gesättigten oder ungesättigten carbocyclischen Ring mit 3 bis 8 Ring-Kohlenstoffatomen stehen,

gefunden.

Weiterhin wurde gefunden, daß man substituierte Carbonsäureanilide der Formel (I) erhält, wenn man

a) Anilin-Derivate der Formel

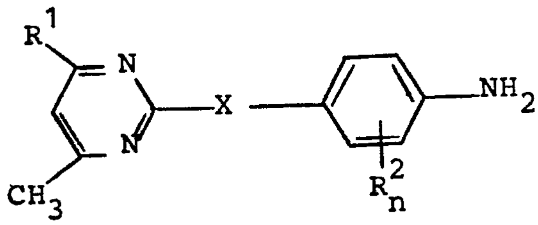

(II)

in welcher

$R^1$, $R^2$, X und n die oben angegebene Bedeutung haben,

entweder

α) mit Säurehalogeniden der Formel

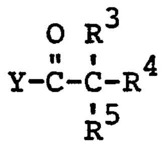

(IIIa)

in welcher

$R^3$, $R^4$ und $R^5$ die oben angegebene Bedeutung haben und

Y für Halogen steht,

gegebenenfalls in Gegenwart eines Verdünnungsmittels und gegebenenfalls in Gegenwart eines Säurebindemittels umsetzt,

oder

ß) mit symmetrischen Carbonsäureanhydriden der Formel

$$O\left(CO-\overset{\displaystyle R^3}{\underset{\displaystyle R^5}{\overset{|}{\underset{|}{C}}}}-R^4\right)_2 \qquad \text{(IIIb)}$$

in welcher

$R^3$,$R^4$ und $R^5$ die oben angegebene Bedeutung haben,

gegebenenfalls in Gegenwart eines Verdünnungsmittels umsetzt,

oder

γ) mit asymmetrischen Säureanhydriden der Formel

$$R-O-\overset{\displaystyle O}{\overset{\|}{C}}-O-\overset{\displaystyle O}{\overset{\|}{C}}-\overset{\displaystyle R^3}{\underset{\displaystyle R^5}{\overset{|}{\underset{|}{C}}}}-R^4 \qquad \text{(III c)}$$

in welcher

$R^3$,$R^4$ und $R^5$ die oben angegebene Bedeutung haben und

R für Alkyl mit 1 bis 4 Kohlenstoffatomen oder Phenyl steht,

gegebenenfalls in Gegenwart eines Verdünnungsmittels umsetzt,

oder

δ) mit Verbindungen der Formel

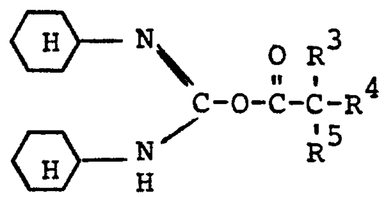

(III d)

in welcher

$R^3$, $R^4$ und $R^5$ die oben angegebene Bedeutung haben,

gegebenenfalls in Gegenwart eines Verdünnungsmittels umsetzt,

oder

b) Pyrimidin-Derivate der Formel

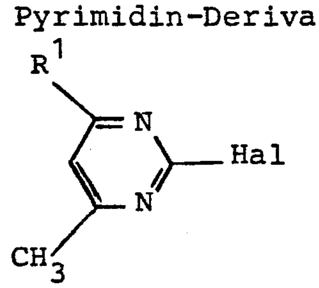

(IV)

in welcher

$R^1$ die oben angegebene Bedeutung hat und

Hal für Halogen steht,

mit Acylanilin-Derivaten der Formel

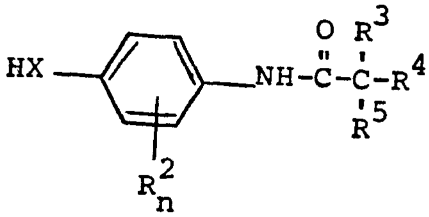

(V)

in welcher

$R^2$, $R^3$, $R^4$, $R^5$, X und n die oben angegebene Bedeutung haben,

in Gegenwart eines Verdünnungsmittels umsetzt.

Schließlich wurde gefunden, daß sich die neuen substituierten Carbonsäureanilide der Formel (I) durch hervorragende herbizide Wirksamkeit auszeichnen.

Überraschenderweise besitzen die erfindungsgemäßen substituierten Carbonsäureanilide der Formel (I) wesentlich bessere herbizide Eigenschaften als die konstitutionell ähnlichsten vorbekannten Stoffe. So lassen sich die erfindungsgemäßen Carbonsäureanilide der Formel (I) wesentlich besser zur Unkrautbekämpfung verwenden als das 2-Phenoxy-4,6-dimethyl-pyrimidin, welches ein strukturell ähnlicher vorbekannter Wirkstoff gleicher Wirkungsart ist.

Die erfindungsgemäßen substituierten Carbonsäureanilide sind durch die Formel (I) allgemein definiert. In dieser Formel steht X für Sauerstoff oder Schwefel. Der Rest $R^1$ steht vorzugsweise für Wasserstoff, Fluor, Chlor,

Brom, geradkettiges oder verzweigtes Alkyl mit 1 bis 4 Kohlenstoffatomen, Trifluormethyl, gegebenenfalls durch Chlor, Trifluormethyl und/oder Methyl substituiertes Phenyl, Alkoxy mit 1 bis 4 Kohlenstoffatomen oder Alkylmercapto mit 1 bis 4 Kohlenstoffatomen. $R^2$ steht vorzugsweise für Chlor, Brom oder Methyl. Der Index n steht vorzugsweise für 0 oder 1. $R^3$ steht vorzugsweise für Wasserstoff, Fluor, Chlor, Brom, gegebenenfalls durch Fluor, Chlor und/oder Brom substituiertes Alkyl mit 1 bis 4 Kohlenstoffatomen, gegebenenfalls durch Fluor, Chlor, Brom, Trifluormethyl, Methoxy und/oder Methyl substituiertes Phenyl, gegebenenfalls durch Fluor, Chlor, Brom, Trifluormethyl, Methoxy und/oder Methyl substituiertes Benzyl oder für die Reste $-OR^6$ oder $-SO_m-R^6$, worin $R^6$ vorzugsweise für gegebenenfalls durch Fluor, Chlor und/oder Brom substituiertes Alkyl mit 1 bis 4 Kohlenstoffatomen oder für gegebenenfalls durch Fluor, Chlor, Brom und/oder Alkyl mit 1 bis 4 Kohlenstoffatomen substituiertes Phenyl steht und der Index m für 0, 1 oder 2 steht. $R^4$ und $R^5$ stehen unabhängig voneinander vorzugsweise für Fluor, Chlor, Brom oder gegebenenfalls durch Fluor, Chlor und/oder Brom substituiertes Alkyl mit 1 bis 4 Kohlenstoffatomen. Außerdem stehen $R^4$ und $R^5$ auch gemeinsam mit dem angrenzenden Kohlenstoffatom vorzugsweise für einen gegebenenfalls durch Fluor, Chlor und/oder Alkyl mit 1 bis 4 Kohlenstoffatomen substituierten gesättigten oder ungesättigten carbocyclischen Ring mit 3 bis 7 Ring -Kohlenstoffatomen.

Eine besonders bevorzugte Gruppe erfindungsgemäßer

Stoffe sind diejenigen substituierten Carbonsäureanilide der Formel (I), in denen

X für Sauerstoff oder Schwefel steht,

$R^1$ für Wasserstoff, Alkyl mit 1 bis 4 Kohlenstoffatomen, Alkoxy mit 1 bis 4 Kohlenstoffatomen oder Trifluormethyl steht,

$R^2$ für Chlor, Brom oder Methyl steht,

n für 0 oder 1 steht,

$R^3$ für Fluor, Chlor, Brom, gegebenenfalls durch Fluor und/oder Chlor substituiertes Alkyl mit 1 bis 4 kohlenstoffatomen, gegebenenfalls durch Fluor, Chlor und/oder Methyl substituiertes Phenyl, gegebenenfalls durch Fluor, Chlor und/oder Methyl substituiertes Benzyl oder für die Reste $-OR^6$ oder $-SO_mR^6$ steht, worin

$R^6$ für gegebenenfalls durch Fluor und/oder Chlor substituiertes Alkyl mit 1 bis 4 Kohlenstoffatomen oder für gegebenenfalls durch Fluor, Chlor und/oder Alkyl mit 1 bis 4 Kohlenstoffatomen substituiertes Phenyl steht und

m für 0, 1 oder 2 steht,

und

$R^4$ und $R^5$ unabhängig voneinander für Fluor, Chlor oder gegebenenfalls durch Fluor und/oder Chlor substituiertes Alkyl mit 1 bis 4 Kohlenstoffatomen stehen

oder

$R^4$ und $R^5$ gemeinsam mit dem angrenzenden Kohlenstoffatom für einen gegebenenfalls durch Fluor, Chlor, Methyl, Ethyl, n-Propyl, iso-Propyl, n-Butyl, iso-Butyl, sek.-Butyl und/oder tert.-Butyl substituierten gesättigten oder ungesättigten carbocyclischen Ring mit 3 bis 7 Ring-Kohlenstoffatomen stehen.

Als Beispiele für substituierte Carbonsäureanilide der Formel (I) seien die in der folgenden Tabelle 1 aufgeführten Stoffe genannt.

Tabelle 1

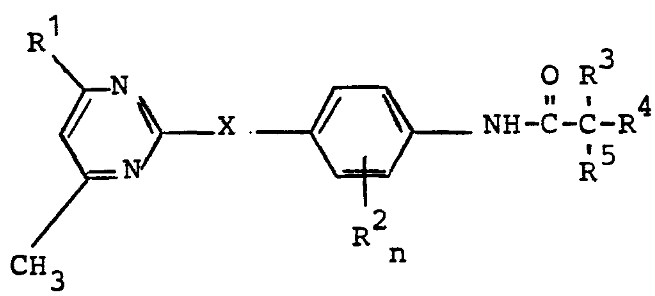

(I)

| $R^1$ | X | $R^2_n$ | $R^3$ | $R^4$ | $R^5$ |
|---|---|---|---|---|---|
| H | O | H | $C_2H_5$ | $CH_3$ | $CH_3$ |
| Cl | O | 2-$CH_3$ | $C_2H_5$ | $CH_3$ | $CH_3$ |
| $CH_3$ | O | 2-$CH_3$ | $C_2H_5$ | $CH_3$ | $CH_3$ |
| $OCH_3$ | O | H | $C_2H_5$ | $CH_3$ | $CH_3$ |
| $CH_3$ | O | H | n-$C_3H_7$ | $CH_3$ | $CH_3$ |
| $CH_3$ | S | H | n-$C_3H_7$ | $CH_3$ | $CH_3$ |
| H | O | H | $OCH_3$ | $CH_3$ | $CH_3$ |
| Cl | O | H | $OCH_3$ | $CH_3$ | $CH_3$ |
| $CH_3$ | O | H | $OCH_3$ | $CH_3$ | $CH_3$ |
| $OCH_3$ | O | H | $OCH_3$ | $CH_3$ | $CH_3$ |
| $CH_3$ | O | H | $SCH_3$ | $CH_3$ | $CH_3$ |
| $CH_3$ | O | H | SO-$CH_3$ | $CH_3$ | $CH_3$ |
| $CH_3$ | O | H | $SO_2$-$CH_3$ | $CH_3$ | $CH_3$ |
| $CH_3$ | O | 2-$CH_3$ | 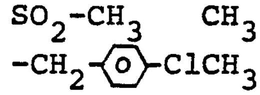 | $CH_3$ | $CH_3$ |
| $CF_3$ | O | H | $CH_3$ | $CH_3$ | $CH_3$ |

Verwendet man 4-(4,6-Dimethyl-pyrimidyl-2-oxy)-anilin und Pivalsäurechlorid als Ausgangsstoffe, so kann der Verlauf des erfindungsgemäßen Verfahrens (a, Variante α) durch das folgende Formelschema wiedergegeben werden:

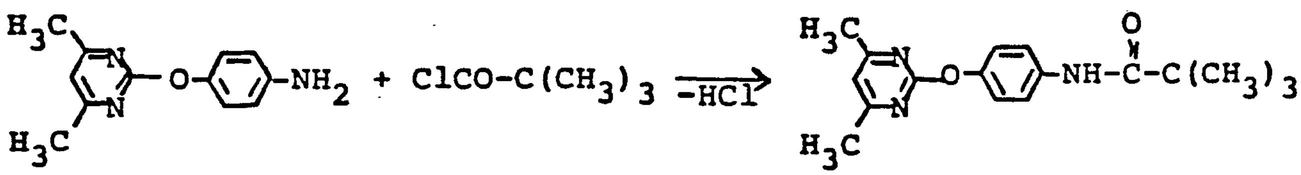


Verwendet man 4-(4-Methyl-pyrimidyl-2-mercapto)-anilin und Isobuttersäureanhydrid als Ausgangsstoffe, so kann der Verlauf des erfindungsgemäßen Verfahrens (a, Variante ß) durch das folgende Formelschema wiedergegeben werden:

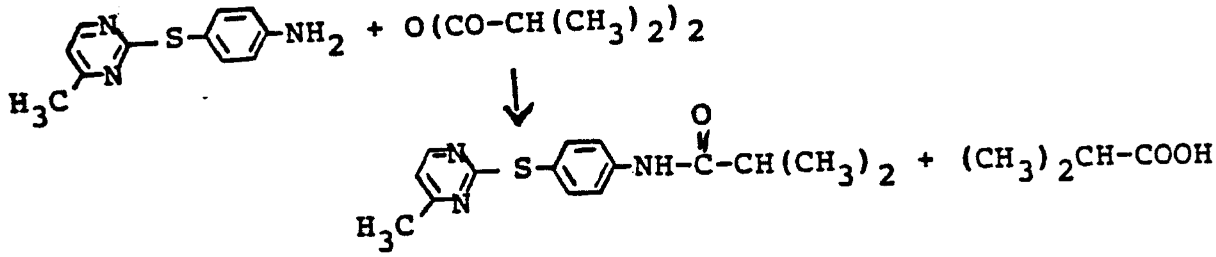

Verwendet man 4-(4,6-Dimethyl-pyrimidyl-2-mercapto)-anilin und α-Methoxy-isobuttersäure-kohlensäure-ethylester-anhydrid als Ausgangsstoffe, so kann der Verlauf des erfindungsgemäßen Verfahrens (a, Variante γ) durch das folgende Formelschema wiedergegeben werden:

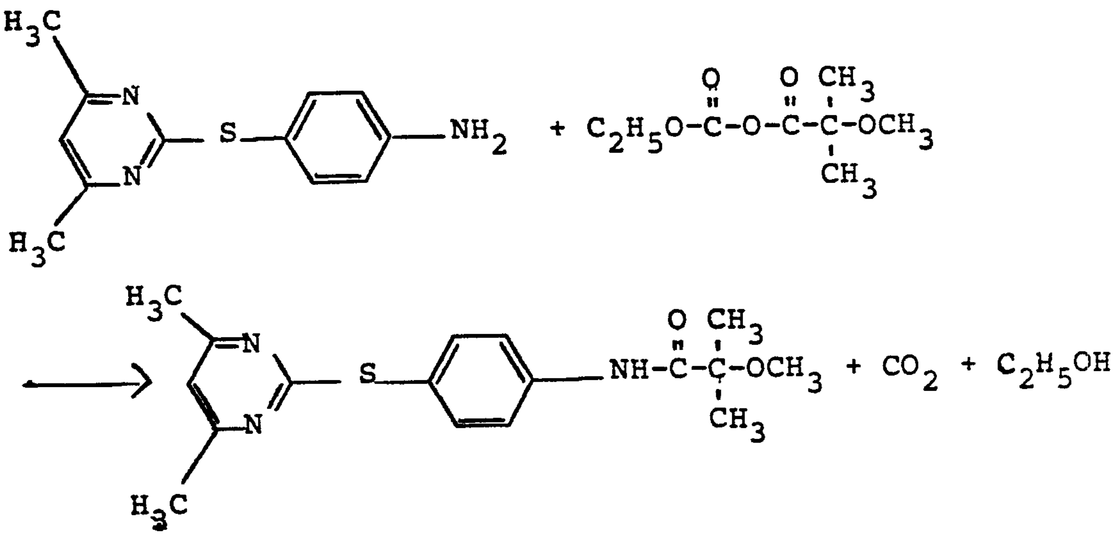

Verwendet man 4-(4,6-Dimethyl-pyrimidyl-2-oxy)-anilin und O-Pivaloyloxy-dicyclohexyl-isoharnstoff als Ausgangsstoffe, so kann der Verlauf des erfindungsgemäßen Verfahrens (a, Variante δ) durch das folgende Formelschema wiedergegeben werden:

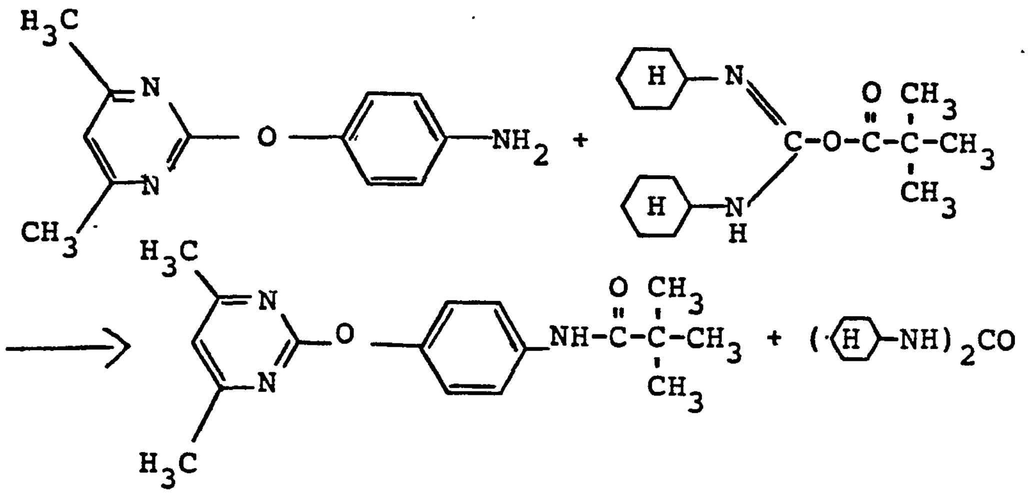

Verwendet man 2-Chlor-4,6-dimethyl-pyrimidin und 4-Pivaloylamino-phenol als Ausgangsstoffe, so kann der Verlauf des erfindungsgemäßen Verfahrens (b) durch das folgende Formelschema wiedergegeben werden:

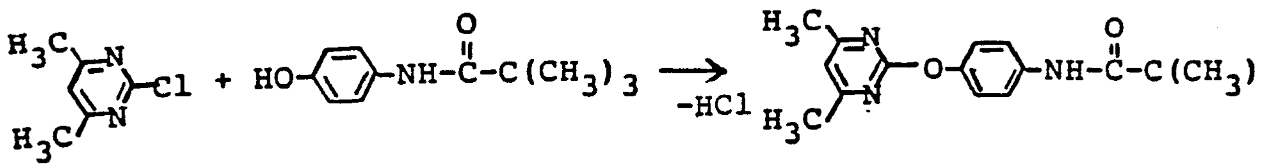


Die bei dem erfindungsgemäßen Verfahren (a) als Ausgangstoffe benötigten Anilin-Derivate sind durch die Formel (II) allgemein definiert. In dieser Formel haben $R^1$,$R^2$, X und n vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Anilin-Derivate der Formel (II) sind teilweise bekannt. Sie lassen sich herstellen, indem man

c) Pyrimidin-Derivate der Formel

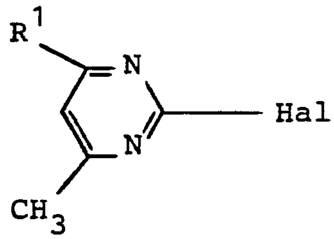

(IV)

in welcher

$R^1$ und Hal die oben angegebene Bedeutung haben,

mit 4-Amino-(thio)-phenolen der Formel

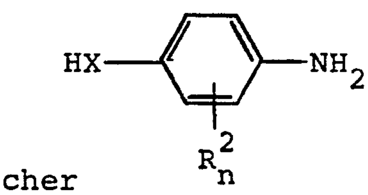

(VI)

in welcher

$R^2$, X und n die oben angegebene Bedeutung haben,

in Gegenwart eines Säurebindemittels sowie gegebenenfalls in Gegenwart eines Verdünnungsmittel umsetzt,

oder

d) 2-(4-Nitro-(thio)-Phenoxy)-pyrimidin-Derivate der Formel

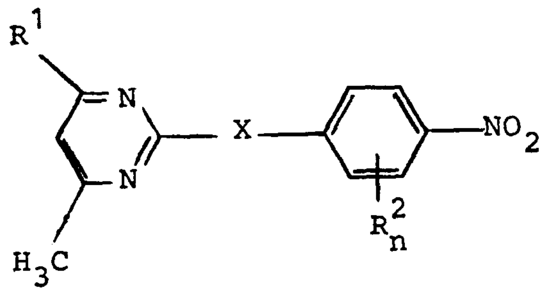

(VII)

in welcher

$R^1$, $R^2$, X und n die oben angegebene Bedeutung haben,

gegebenenfalls in Gegenwart eines Verdünnungsmittels nach üblichen Methoden reduziert.

Die bei dem obigen Verfahren (c) als Ausgangsstoffe benötigten Pyrimidin-Derivate sind durch die Formel (IV) definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Rest genannt wurden. Hal steht vorzugsweise für Fluor, Chlor oder Brom.

Als Beispiele für Pyrimidin-Derivate der Formel (IV) seien genannt:

2-Chlor-4-methyl-pyrimidin
2-Brom-4-methyl-pyrimidin
2,4-Dichlor-6-methyl-pyrimidin
2-Chlor-4-methoxy-6-methylpyrimidin

2-Chlor-4-methylmercapto-6-methyl-pyrimidin

2-Chlor-4,6-dimethyl-pyrimidin
2-Fluor-4,6-dimethyl-pyrimidin
2-Chlor-4-methyl-6-propyl-pyrimidin
2-Chlor-4-methyl-6-isopropyl-pyrimidin
2-Chlor-4-methyl-6-trifluormethyl-pyrimidin
2-Chlor-4-methyl-6-phenyl-pyrimidin
2-Chlor-4-methyl-6-(4-chlor-phenyl)-pyrimidin
2-Chlor-4-methyl-6-(4-methyl-phenyl)-pyrimidin
2-Chlor-4-methyl-6-(3-trifluormethyl-phenyl)-pyrimidin

Die Pyrimidin-Derivate der Formel (IV) sind bekannt oder lassen sich nach im Prinzip bekannten Methoden in einfacher Weise herstellen. So erhält man Pyrimidin-Derivate der Formel (IV) zum Beispiel dadurch, daß man 2-Hydroxy-pyrimidin-Derivate (Dihydro-pyrimidon-2-Derivate) mit anorganischen Säurehalogeniden, wie zum Beispiel Phosphoroxychlorid oder Phosphorpentachlorid, umsetzt, oder auch dadurch, daß man entsprechende 2-Amino-pyrimidin-Derivate mit Salpetriger Säure in Gegenwart von Halogenwasserstoffsäuren umsetzt.

Die bei dem Verfahren (c) weiterhin als Ausgangsstoffe benötigten 4-Amino-(thio)-phenole sind durch die Formel (VI) definiert. In dieser Formel haben $R^2$, X und n vorzugsweise diejenigen Bedeutungen, die bereits im Zusammenhang mit der Beschreibung der erfindungsgemäßen Stoffe der Formel (I) vorzugsweise für diese Reste bzw. für diesen Index genannt wurden.

Als Beispiele für 4-Amino-(thio)-phenole der Formel

(VI) seien genannt:

4-Amino-phenol
2-Chlor-4-amino-phenol
3-Chlor-4-amino-phenol
2,5-Dichlor-4-amino-phenol
2,6-Dichlor-4-amino-phenol
2-Methyl-4-amino-phenol
3-Methyl-4-amino-phenol
2-Chlor-5-methyl-4-amino-phenol
4-Amino-thiophenol
2-Chlor-4-amino-thiophenol
3-Chlor-4-amino-thiophenol
2-Methyl-4-amino-thiophenol
3-Methyl-4-amino-thiophenol

Die 4-Amino-(thio)-phenole der Formel (VI) sind bekannt oder lassen sich nach im Prinzip bekannten Methoden in einfacher Weise herstellen.

Als Säurebindemittel können bei der Durchführung des Verfahrens (c) alle üblicherweise für derartige Umsetzungen verwendbaren Säureakzeptoren verwendet werden. Vorzugsweise verwendbar sind Alkali- und Erdalkalioxide, -hydroxide und -carbonate, wie Natriumhydroxid, Kaliumhydroxid, Calciumoxid, Natriumcarbonat und Kaliumcarbonat, ferner Alkali-alkoholate, -amide und -hydride, wie zum Beispiel Natrium-methylat, Natriumethylat, Kalium-tert.-butylat, Natrium-amid und Natrium-hydrid.

Als Verdünnungsmittel können bei der Durchführung des Verfahrens (c) alle üblichen inerten organischen Solventien verwendet werden. Vorzugsweise in Frage kommen Kohlenwasserstoffe, wie Benzin, Toluol und Xylol, ferner Ether, wie Dioxan, Glykoldimethylether und Diglykoldimethylether, außerdem Nitrile, wie Acetonitril, und auch stark polare Solventien, wie Dimethylsulfoxid, Sulfolan und Dimethylformamid.

Die Reaktionstemperaturen können bei der Durchführung des Verfahrens (c) innerhalb eines größeren Bereiches variiert werden. Im allgemeinen arbeitet man bei Temperaturen zwischen 0°C und 200°C, vorzugsweise zwischen 50°C und 150°C.

Die Umsetzung nach dem Verfahren (c) wird im allgemeinen unter Normaldruck vorgenommen.

Bei der Durchführung des Verfahrens (c) setzt man die Ausgangsstoffe der Formeln (IV) und(VI) im allgemeinen in angenähert äquimolaren Mengen um. Es ist jedoch auch möglich, die eine oder andere Komponente in einem größeren Überschuß zu verwenden. Die Aufarbeitung erfolgt nach üblichen Methoden.

Die bei dem Verfahren (d) als Ausgangsstoffe benötigten 2-(4-Nitro-(thio)-phenoxy)-pyrimidin-Derivate sind durch die Formel (VII) definiert. In dieser Formel haben $R^1$, $R^2$, X und n vorzugsweise diejenigen Bedeutungen, die bereits im Zusammenhang mit der Beschreibung

der erfindungsgemäßen Stoffe der Formel (I) vorzugsweise für diese Reste bzw. für diesen Index genannt wurden.

Die Verbindungen der Formel (VII) sind bekannt oder lassen sich nach im Prinzip bekannten Methoden in einfacher Weise herstellen. So erhält man Verbindungen der Formel (VII) zum Beispiel dadurch, daß man Pyrimidin-Derivate der Formel

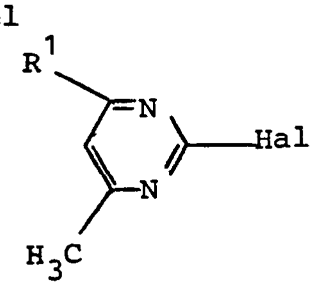

(IV)

in welcher

$R^1$ und Hal die oben angegebene Bedeutung haben,

mit 4-Nitro-(thio)-phenolen der Formel

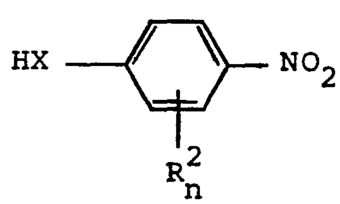

(IX)

in welcher

$R^2$, X und n die oben angegebene Bedeutung haben,

in Gegenwart eines Säurebindemittels und gegebenenfalls in Gegenwart eines Verdünnungsmittels bei Temperaturen zwischen 0°C und 200°C, vorzugsweise zwischen 50°C und 150°C, umsetzt. Als Säurebindemittel und Verdünnungsmittel kommen hierbei vorzugsweise diejenigen Stoffe in Betracht, die bereits im Zusammenhang mit dem Verfahren (c) als vorzugsweise verwendbare Säureakzeptoren und Solventien genannt wurden.

Als Reduktionsmittel kommen bei dem Verfahren (d) alle diejenigen Stoffe in Frage, die üblicherweise zur Reduktion aromatischer Nitroverbindungen eingesetzt werden. Vorzugsweise verwendbar sind elementare Metalle, wie Eisen, Zink und Zinn, ferner Metallverbindungen in niederen Wertigkeitsstufen, wie Eisen (II)- und Zinn (II)-Salze, und außerdem Nichtmetall-Verbindungen in niederen Wertigkeitsstufen, wie zum Beispiel Salze des Schwefelwasserstoffes, Alkalisulfite und Alkalidithionite. Im übrigen kann die Reduktion auch durch katalytische Hydrierung mit Wasserstoff in Gegenwart eines Katalysators, wie zum Beispiel Raney-Nickel, erfolgen.

Als Verdünnungsmittel kommen bei dem Verfahren (d) alle üblichen für derartige Reduktionen geeigneten organischen Solventien in Betracht. Die Reaktionstemperaturen können innerhalb eines größeren Bereiches variiert werden. Sie entsprechen den Temperaturen, die bei analogen Reaktionen angewandt werden.

Die Durchführung der Reduktion nach dem Verfahren (d) und die Aufarbeitung des anfallenden Reaktionsgemisches erfolgen nach üblichen Methoden.

Die bei dem erfindungsgemäßen Verfahren (a, Variante $\alpha$ )

als Reaktionskomponenten benötigten Säurehalogenide sind durch die Formel (III a) eindeutig definiert. In dieser Formel haben $R^3$, $R^4$ und $R^5$ vorzugsweise diejenigen Bedeutungen, die bereits im Zusammenhang mit der Beschreibung der erfindungsgemäßen Stoffe der Formel (I) vorzugsweise für diese Reste genannt wurden. Y steht vorzugsweise für Fluor, Chlor und Brom.

Als Beispiele für Säurehalogenide der Formel (III a) seien die Chloride der folgenden Säuren genannt:

Trichloressigsäure
Trifluoressigsäure
α,α-Dichlorpropionsäure
Isobuttersäure
α-Chlor-isobuttersäure
α-Brom-isobuttersäure
α-Methoxy-isobuttersäure
α-Phenoxy-isobuttersäure
α-(4-Chlor-phenoxy)-isobuttersäure
α-(2-Methyl-4-chlor-phenoxy)-isobuttersäure
α-Methylmercapto-isobuttersäure
α-Methylsulfonyl-isobuttersäure
α-Methyl-buttersäure
Pivalinsäure
β-Fluor-pivalinsäure
β-Chlor-pivalinsäure
β,β'-Difluor-pivalinsäure
β,β'-Dichlor-pivalinsäure
β,β'β"-Trifluor-pivalinsäure
β,β'β"-Trichlor-pivalinsäure

α,α-Dimethyl-valeriansäure
α-Methyl-α-ethyl-buttersäure
α,α-Dimethyl-phenylessigsäure
α,α-Dimethyl-(4-chlor-phenyl)-essigsäure
α,α-Dimethyl-(3,4-dichlor-phenyl)-essigsäure
α,α-Dimethyl-(3-trifluormethyl-phenyl)-essigsäure
α-Benzyl-isobuttersäure
α-(4-Chlor-benzyl)-isobuttersäure
α-(4-Methoxy-benzyl)-isobuttersäure
Cyclopropan-carbonsäure
1-Methyl-cyclopropan-carbonsäure
2,2-Dichlor-1-methyl-cyclopropan-carbonsäure
Cyclopentan-carbonsäure
1-Methyl-cyclopentan-carbonsäure
Cyclohexan-carbonsäure
1-Methyl-cyclohexan-carbonsäure
1-Methyl-4-isopropyl-cyclohexan-carbonsäure

Die Säurehalogenide der Formel (III a) sind bekannt oder lassen sich nach im Prinzip bekannten Methoden in einfacher Weise herstellen.

Als Säurebindemittel kommen bei der Umsetzung nach dem erfindungsgemäßen Verfahren (a, Variante α) alle üblichen Säureakzeptoren in Betracht. Vorzugsweise verwendbar sind tertiäre Amine, wie Triethylamin, Pyridin und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat. Es ist auch möglich,

die jeweiligen Anilin-Derivate der Formel (II) gleichzeitig als Säurebindemittel zu verwenden. Dazu muß die betreffende Anilin-Verbindung dann zumindest in solcher Menge eingesetzt werden, daß der freiwerdende Halogenwasserstoff gebunden werden kann.

Als Verdünnungsmittel können bei dem erfindungsgemäßen Verfahren (a, Variante $\alpha$) alle gegenüber Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methyl-isopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Die Reaktionstemperaturen können bei der Durchführung des erfindungsgemäßen Verfahrens (a, Variante $\alpha$) innerhalb eines größeren Bereiches variiert werden. Arbeitet man ohne Lösungsmittel und Säurebindemittel, so geht man im allgemeinen so vor, daß man die Komponenten zunächst bei Temperaturen zwischen -20°C und +20°C reagieren läßt und danach auf Temperaturen zwischen 70 und 200°C erhitzt. Arbeitet man in Gegenwart eines Verdünnungsmittels und eines Säurebindemittels, so liegen die

Reaktionstemperaturen im allgemeinen zwischen -20°C und +100°C, vorzugsweise zwischen 0°C und 50°C.

Das erfindungsgemäße Verfahren (a, Variante α) wird im allgemeinen unter Normaldruck durchgeführt.

Bei der Durchführuhg des erfindungsgemäßen Verfahrens (a, Variante α) werden die Ausgangsstoffe der Formeln (II) und (III a) im allgemeinen in angenähert äquivalenten Mengen verwendet. Es ist jedoch auch möglich, die eine oder andere Komponente in einem größeren Überschuß einzusetzen. Die Aufarbeitung erfolgt danach üblichen Methoden. Im allgemeinen geht man so vor, daß man ausgefallene Salze entfernt und das verbleibende Reaktionsgemisch durch Abziehen des Verdünnungsmittels einengt. Arbeitet man in Gegenwart von Wasser oder von mit Wasser mischbaren Solventien, so kann man auch so verfahren, daß man das Reaktionsgemisch mit Wasser verdünnt, das entstehende Gemisch abgesaugt oder mit einem Wasser wenig mischbaren organischen Lösungsmittel extrahiert, die organische Phase wäscht, einengt und den verbleibenden Rückstand gegebenenfalls üblichen Reinigungsverfahren unterwirft.

Die bei dem erfindungsgemäßen Verfahren (a, Variante ß) als Reaktionskomponenten benötigten symmetrischen Carbonsäure-anhydride sind durch die Formel (III b) eindeutig definiert. In dieser Formel haben $R^3$, $R^4$ und $R^5$ vorzugsweise diejenigen Bedeutungen, die bereits im Zusammenhang mit der Beschreibung der erfindungsgemäßen

Stoffe der Formel (I) vorzugsweise für diese Reste genannt wurden.

Die symmetrischen Carbonsäureanhydride der Formel (III b) sind bekannt oder lassen sich nach im Prinzip bekannten Methoden in einfacher Weise herstellen.

Als Verdünnungsmittel können bei der Durchführung des erfindungsgemäßen Verfahrens (a, Variante ß) vorzugsweise diejenigen Verdünnungsmittel verwendet werden, die auch bei dem Verfahren (a, Variante α) vorzugsweise in Betracht kommen. Im übrigen kann auch ein im Überschuß eingesetztes Carbonsäureanhydrid der Formel (III b) gleichzeitig als Verdünnungsmittel fungieren.

Die Reaktionstemperaturen können auch bei dem erfindungsgemäßen Verfahren (a, Variante ß) innerhalb eines größeren Bereiches variiert werden. Im allgemeinen arbeitet man bei Temperaturen zwischen -20°C und +150°C, vorzugsweise zwischen 0 und 100°C.

Das erfindungsgemäße Verfahren (a, Variante ß) wird im allgemeinen unter Normaldruck durchgeführt.

Bei der Durchführung des erfindungsgemäßen Verfahrens (a, Variante ß) werden die Ausgangsstoffe der Formeln (II) und (III b) im allgemeinen in angenähert äquivalenten Mengen verwendet. Es ist jedoch auch möglich, das Carbonsäureanhydrid in einem größeren Überschuß einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Die bei dem erfindungsgemäßen Verfahren (a, Variante γ) als Reaktionskomponenten benötigten asymmetrischen Säureanhydride sind durch die Formel (III c) eindeutig definiert. In dieser Formel haben $R^3$, $R^4$ und $R^5$ vorzugsweise diejenigen Bedeutungen, die bereits im Zusammenhang mit der Beschreibung der erfindungsgemäßen Stoffe der Formel (I) vorzugsweise für diese Reste genannt wurden. R steht vorzugsweise für Alkyl mit 1 oder 2 Kohlenstoffatomen oder für Phenyl.

Die asymmetrischen Säureanhydride der Formel (III c) sind bekannt oder lassen sich nach im Prinzip bekannten Methoden in einfacher Weise herstellen. So erhält man Verbindungen der Formel (III c) dadurch, daß man Carbonsäuren der Formel

$$HO-\overset{\overset{O}{\|}}{C}-\underset{\underset{R^5}{|}}{\overset{\overset{R^3}{|}}{C}}-R^4 \qquad (III)$$

in welcher

$R^3$, $R^4$ und $R^5$ die oben angegebene Bedeutung haben,

mit Kohlensäure-ester-chloriden der Formel

$$R-O-\overset{\overset{O}{\|}}{C}-Cl \qquad (X)$$

in welcher

R die oben angegebene Bedeutung hat,

in Gegenwart eines Verdünnungsmittels, wie zum Beispiel Methylenchlorid, und in Gegenwart eines Säurebindemittels, wie zum Beispiel Triethylamin, bei Temperaturen zwischen -20°C und +100°C, vorzugsweise zwischen 0 und 50°C, umsetzt. Die asymmetrischen Säureanhydride der Formel (III c) werden im allgemeinen nicht in reiner Form isoliert, sondern in der anfallenden Form, gegebenenfalls nach vorheriger Entfernung von Verdünnungsmittel und/ oder Salzen, weiter verwendet.

Als Verdünnungsmittel können bei der Durchführung des erfindungsgemäßen Verfahrens (a, Variante $\gamma$) vorzugsweise diejenigen Verdünnungsmittel verwendet werden, die auch bei dem Verfahren (a, Variante $\alpha$) vorzugsweise in Betracht kommen. Im übrigen kann auch im Überschuß eingesetztes Säureanhydrid der Formel (III c) gleichzeitig als Verdünnungsmittel fungieren.

Die Reaktionstemperaturen können auch bei dem erfindungsgemäßen Verfahren (a, Variante $\gamma$) innerhalb eines größeren Bereiches variiert werden. Im allgemeinen

arbeitet man bei Tempearaturen zwischen -20°C und +150°C, vorzugsweise zwischen 0 und 100°C.

Das erfindungsgemäße Verfahren (a, Variante γ) wird im allgemeinen unter Normaldruck durchgeführt.

Bei der Durchführung des erfindungsgemäßen Verfahrens (a, Variante γ) werden die Ausgangsstoffe der Formeln (II) und (III c) im allgemeinen in angenähert äquivalenten Mengen verwendet. Es ist jedoch auch möglich, das Säureanhydrid in einem größeren Überschuß einzusetzen. Die Aufarbeitung erfolgt nach üblichen Methoden.

Die bei dem erfindungsgemäßen Verfahren (a, Variante δ) als Reaktionskomponenten benötigten Verbindungen sind durch die Formel (III d) eindeutig definiert. In dieser Formel haben $R^3$, $R^4$ und $R^5$ vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (III d) sind bekannt oder lassen sich nach im Prinzip bekannten Verfahren in einfacher Weise herstellen. So erhält man Verbindungen der Formel (III d) dadurch, daß man Carbonsäuren der Formel

$$HO-\overset{\overset{O}{\|}}{C}-\overset{R^3}{\underset{R^5}{C}}-R^4 \qquad (III)$$

in welcher

$R^3$, $R^4$ und $R^5$ die oben angegebene Bedeutung haben,

mit Dicyclohexylcarbodiimid der Formel

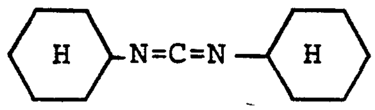

(XI)

umsetzt.

Als Verdünnungsmittel können bei der Durchführung des erfindungsgemäßen Verfahrens (a, Variante δ) vorzugsweise diejenigen Verdünnungsmittel verwendet werden, die auch bei dem Verfahren (a, Variante α) vorzugsweise in Betracht kommen.

Die Reaktionstemperaturen können auch bei dem erfindungsgemäßen Verfahren (a, Variante δ) innerhalb eines größeren Bereiches variiert werden. Im allgemeinen arbeitet man bei Temperaturen zwischen -20°C und +150°C, vorzugsweise zwischen 0 und 100°C.

Das erfindungsgemäße Verfahren (a, Variante δ) wird im allgemeinen unter Normaldruck durchgeführt.

Bei der Durchführung des erfindungsgemäßen Verfahrens (a, Variante δ) werden die Ausgangsstoffe der Formeln (II) und (III d) im allgemeinen in angenähert äquivalenten Mengen verwendet. Die Aufarbeitung erfolgt nach üblichen Methoden.

Die bei dem erfindungsgemäßen Verfahren (b) als Ausgangsstoffe benötigten Pyrimidin-Derivate wurden bereits im Zusammenhang mit der Beschreibung des Verfahrens (c) abgehandelt.

Die bei dem erfindungsgemäßen Verfahren (b) weiterhin als Ausgangsstoffe benötigten Acylanilin-Derivate sind durch die Formel (V) eindeutig definiert. In dieser Formel haben $R^2$, $R^3$, $R^4$, $R^5$, X und n vorzugsweise diejenigen Bedeutungen, die bereits im Zusammenhang mit der Beschreibung der Stoffe der Formel (I) vorzugsweise für diese Reste bzw. für diesen Index genannt wurden.

Die Verbindungen der Formel (V) sind bekannt oder lassen sich nach im Prinzip bekannten Methoden in einfacher Weise herstellen. So erhält man Acylanilin-Derivate der Formel (V) zum Beispiel dadurch, daß man 4-Amino-(thio)-phenole der Formel

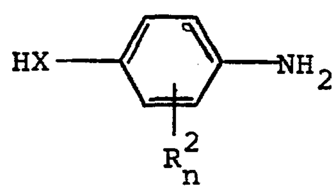

(VI)

in welcher

$R^2$, X und n die oben angegebene Bedeutung haben,

mit Säurehalogeniden der Formel

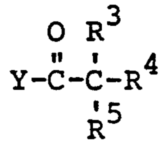

(III a)

in welcher

$R^3$, $R^4$, $R^5$ und Y die oben angegebene Bedeutung haben,

gegebenenfalls in Gegenwart eines Verdünnungsmittels und gegebenenfalls in Gegenwart eines Säurebindemittels umsetzt. Die Reaktionsbedingungen entsprechen dabei denjenigen, die auch bei der Durchführung des Verfahrens (a, Variante α) angewandt werden.

Als Säurebindemittel können bei der Durchführung des erfindungsgemäßen Verfahrens (b) alle üblichen Säureakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, ferner Alkalimetall- und Erdalkalimetallamide und -hydride, wie Natriumamid, Natriumhydrid und Calciumhydrid, und außerdem auch Alkalimetall-alkoholate, wie Natrium-methylat, Natriumethylat und Kalium-tert.-butylat.

Als Verdünnungsmittel können bei dem erfindungsgemäßen Verfahren (b) alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem Nitrile, wie Acetonitril und Propionitril, und weiterhin polare

Lösungsmittel, wie Nitrobenzol, Dimethylsulfoxid, Sulfolan, Dimethylformamid und N-Methyl-pyrrolidon.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0 und 200°C, vorzugsweise zwischen 50 und 150°C.

Das erfindungsgemäße Verfahren (b) wird im allgemeinen unter Normaldruck durchgeführt.

Bei der Durchführung des erfindungsgemäßen Verfahrens (b) setzt man die Reaktionskomponenten der Formeln (IV) und (V) im allgemeinen in etwa äquimolaren Mengen ein. Es ist jedoch auch möglich, die eine oder andere Komponente in einem größeren Überschuß zu verwenden. Außerdem setzt man im allgemeinen auch eine äquimolare Menge an säurebindendem Mittel ein. Es kann jedoch auch von Vorteil sein, das Säurebindemittel in einem bis zu einmolaren Überschuß hinzuzufügen. Im einzelnen geht man im allgemeinen so vor, daß man das säurebindende Mittel zu einer Mischung der Reaktionskomponenten in einem geeigneten Verdünnungsmittel gibt. Man kann aber auch in der Weise verfahren, daß man zunachst aus dem Acylanilin-Derivat der Formel (V) und dem Säurebindemittel ein Salz erzeugt und dieses dann mit einem Pyrimidin-Derivat der Formel (IV) umsetzt. Weiterhin ist es auch möglich, aus dem Acylanilin-Derivat der Formel (V) mit

einem Säurebindemittel zunächst separat ein Salz herzustellen, dieses dann zu isolieren und anschließend in Gegenwart eines geeigneten Verdünnungsmittels ohne weitere Zugabe eines Säurebindemittels mit einem Pyrimidin-Derivat der Formel (IV) umzusetzen. -Die Aufarbeitung erfolgt jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich besonders gut zur selektiven Bekämpfung mono- und dikotyler Unkräuter in monokotylen Kulturen, wie zum Beispiel Mais und Getreide.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe lassen sich mit besonderem Vorteil zur Bekämpfung von Pyricularia oryzae bei Reis, gegen Botrytis und zur Bekämpfung von Bohnenrost verwenden.

Im übrigen zeichnen sich die erfindungsgemäßen Stoffe auch durch eine insektizide Wirksamkeit aus. Sie besitzen dabei eine gute wurzelsystemische Wirksamkeit.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z. B. N-(2-Benzthiazolyl)-N,N'-dimethyl-harnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on, 2-Chlor-4-ethylamino-6-isopropyl-amino-1,3,5-triazin, das R-Enantiomere des 2-[4-(3,5-Dichlor-pyridin-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-4-chlor-phenoxy)-propionsäure, 3,5-Dijod-4-hydroxy-benzonitril, 3,5-Dibrom-4-hydroxy-benzonitril sowie Diphenylether und Phenylpyridazine, wie zum Beispiel Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können bei einer Verwendung als Herbizide sowohl vor als auch nach dem Auflaufen der Pflanzen appliziert werden. Sie können auch vor der Saat in den Boden eingearbeitet werden.

Die angewandte Wirkstoffmenge kann beim Einsatz der erfindungsgemäßen Stoffe als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen

Wirkstoffmengen von 0,001 bis 50 g je kg Saatgut, vorzugsweise 0,01 bis 10 g, benötigt. Bei Behandlung des Bodens sind Wirkstoffkonzentrationen von 0.00001 bis 0,1 Gew.-%, vorzugsweise von 0,0001 bis 0,02 %, am Wirkungsort erforderlich.

Die Herstellung und die Verwendung der erfindungsgemäßen Wirkstoffe geht aus den nachfolgenden Beispielen hervor.

Herstellungsbeispiele

Beispiel 1

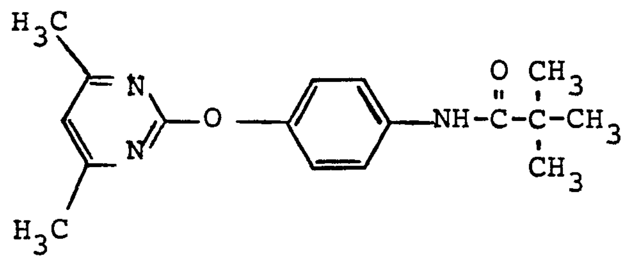

(I-1)

(Verfahren a)

21,5 g (0,1 Mol) 2-(4-Amino-phenoxy)-4,6-dimethyl-pyrimidin wurden in 150 ml Tetrahydrofuran gelöst. Man gab 10,1 g (0,1Mol) Triethylamin hinzu und tropfte dann bei einer Temperatur von 10 bis 15°C unter Rühren 12 g (0,1 Mol) Pivalsäurechlorid ein. Das Gemisch wurde 2 Stunden bei Raumtemperatur nachgerührt. Danach wurde aufgearbeitet, indem man das abgeschiedene Festprodukt absaugte und das Filtrat unter vermindertem Druck eindampfte. Es verblieben 27,5 g (92 % der Theorie) an 2-(4-Pivaloylamino-phenoxy)-4,6-dimethyl-pyrimidin in Form eines Festproduktes.

Schmelzpunkt: 190-191°C (nach Umkristallisation aus Essigester)

Beispiel 2

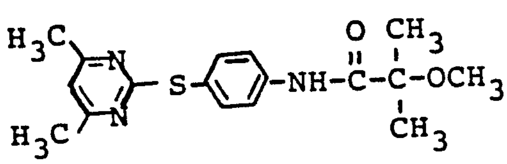

(I-2)

11,8 g (0,1 Mol) α-Methoxy-isobuttersäure wurden in 150 ml Tetrahydrofuran gelöst und mit 10,1 g (0,1 Mol) Triethylamin versetzt. Hierzu tropfte man bei 10 - 15°C unter Rühren 10,85 g (0,1 Mol) Kohlensäureethylesterchlorid. Die Mischung wurde 2 Stunden bei Raumtemperatur nachgerührt und anschließend portionsweise mit 23,1 g (0,1 Mol) 2-(4-Amino-phenylmercapto)-4,6-dimethyl-pyrimidin versetzt. Man rührte 1 Stunde bei Raumtemperatur nach und kochte noch 2 Stunden unter Rückfluß. Das Reaktionsgemisch wurde abgekühlt und dann in 1 Liter schwach mit Salzsäure angesäuertes Wasser eingerührt. Die abgeschiedenen Kristalle wurden abgesaugt, mit Wasser gewaschen und an der Luft getrocknet. Man erhielt 27,4 g (83 % der Theorie) 2-(4-(α-Methoxy-isobutyrylamino)-phenylmercapto)-4,6-dimethyl-pyrimidin.

Schmelzpunkt: 123 - 125°C (nach Umkristallisation aus Waschbenzin)

Beispiel 3

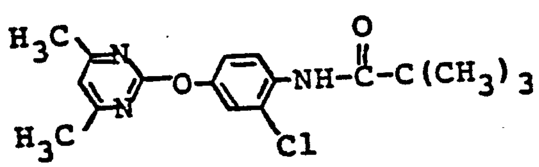

(I-3)

(Verfahren b)

22,8 g (0,1 Mol) 3-Chlor-4-pivaloylamino-phenol wurden in 150 ml Dimethylsulfoxid gelöst. Hierzu gab man bei Raumtemperatur unter Rühren portionsweise 5,6 g (0,1 Mol)

pulverisiertes Kaliumhydroxid. Man rührte 30 Minuten bei Raumtemperatur nach und fügte dann portionsweise 14,25 g (0,1 Mol) 2-Chlor-4,6-dimethyl-pyrimidin hinzu. Die Mischung wurde innerhalb einer Stunde auf 120°C erhitzt und noch 5 Stunden bei dieser Temperatur nachgerührt. Anschließend wurde das Gemisch abgekühlt und in 1 Liter Wasser eingegossen. Die Kristalle wurden abgesaugt und an der Luft getrocknet. Man erhielt 25,5 g (76,5 % der Theorie) 2-(3-Chlor-4-pivaloylamino-phenoxy)-4,6-dimethylpyrimidin.

Schmelzpunkt: 83 - 85°C

Nach den in den vorausgehenden Beispielen und in der Beschreibung angegebenen Methoden wurden auch die in der folgenden Tabelle formelmäßig aufgeführten Stoffe der Formel (I) hergestellt.

Tabelle 2

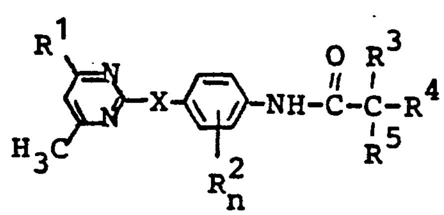

(I)

Tabelle 2

| Bsp.-Nr. | $R^1$ | X | $R^2$ | $R^3$ | $R^4$ | $R^5$ | Schmelzpunkt (°C) |
|---|---|---|---|---|---|---|---|
| 4 | H | O | H | $CH_3$ | $CH_3$ | $CH_3$ | 161-163 |
| 5 | H | O | H | $CH_2F$ | $CH_3$ | $CH_3$ | 132-134 |
| 6 | H | O | H | $CH_2Cl$ | $CH_3$ | $CH_3$ | 148-150 |
| 7 | H | O | H | $CH_2F$ | $CH_2F$ | $CH_2F$ | 127-129 |
| 8 | H | S | H | $CH_3$ | $CH_3$ | $CH_3$ | 150-152 |
| 9 | H | S | H | $CH_2Cl$ | $CH_3$ | $CH_3$ | 120-121 |
| 10 | H | S | H | $CH_3$ | $-(CH_2)_4-$ | | 119-121 |
| 11 | Cl | O | H | $CH_3$ | $CH_3$ | $CH_3$ | 179-181 |
| 12 | $OCH_3$ | O | H | $CH_3$ | $CH_3$ | $CH_3$ | 164-166 |
| 13 | $CH_3$ | O | H | H | $CH_3$ | $CH_3$ | 163-165 |
| 14 | $CH_3$ | O | H | H | $CH_3$ | $C_2H_5$ | 142-144 |
| 15 | $CH_3$ | O | H | H | $-(CH_2)_5-$ | | 204-206 |
| 16 | $CH_3$ | O | H | Cl | Cl | Cl | 194-196 |
| 17 | $CH_3$ | O | H | Br | $CH_3$ | $CH_3$ | 168-170 |
| 18 | $CH_3$ | O | H | $OCH_3$ | $CH_3$ | $CH_3$ | 132-134 |
| 19 | $CH_3$ | O | H | $CH_2F$ | $CH_3$ | $CH_3$ | 164-166 |
| 20 | $CH_3$ | O | H | $CH_2Cl$ | $CH_3$ | $CH_3$ | 168-170 |
| 21 | $CH_3$ | O | H | $CH_2F$ | $CH_2F$ | $CH_3$ | 166 |

Tabelle 2 (Fortsetzung)

| Bsp.-Nr. | $R^1$ | X | $R^2$ | $R^3$ | $R^4$ | $R^5$ | Schmelzpunkt (°C) |
|---|---|---|---|---|---|---|---|
| 22 | $CH_3$ | O | H | $CH_2Cl$ | $CH_2Cl$ | $CH_3$ | 181-182 |
| 23 | $CH_3$ | O | H | $CH_2F$ | $CH_2F$ | $CH_2F$ | 166-168 |
| 24 | $CH_3$ | O | H | $CH_2Cl$ | $CH_2Cl$ | $CH_2Cl$ | 101-103 |
| 25 | $CH_3$ | O | H | $C_3H_7$ | $CH_3$ | $CH_3$ | 138-140 |
| 26 | $CH_3$ | O | H | $C_2H_5$ | $C_2H_5$ | $CH_3$ | 158-160 |
| 27 | $CH_3$ | O | H | - 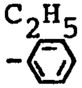 -Cl | $CH_3$ | $CH_3$ | 150-152 |
| 28 | $CH_3$ | O | H | $CH_2$- 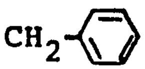 | $CH_3$ | $CH_3$ | 134-136 |
| 29 | $CH_3$ | O | H | $CH_3$ | $-CH_2-CH_2-$ | | 203-205 |
| 30 | $CH_3$ | O | H | $CH_3$ | $-CH_2-CCl_2-$ | | 154-156 |
| 31 | $CH_3$ | O | H | $CH_3$ | $-(CH_2)_4-$ | | 140-142 |
| 32 | $CH_3$ | O | H | $CH_3$ | $-(CH_2)_5-$ | | 139-140 |
| 33 | $CH_3$ | O | H | $CH_3$ | $-(CH_2)_2-CH(CH(CH_3)_2)-(CH_2)_2$ | | 128-130 |
| 34 | $CH_3$ | O | 3-Cl | $CH_3$ | $CH_3$ | $CH_3$ | 205-207 |
| 35 | $CH_3$ | O | 3-Cl | $CH_2Cl$ | $CH_3$ | $CH_3$ | 182-184 |
| 36 | $CH_3$ | O | 3-Cl | $CH_2F$ | $CH_2F$ | $CH_3$ | 178-180 |
| 37 | $CH_3$ | O | 3,5-$Cl_2$ | $CH_3$ | $CH_3$ | $CH_3$ | 250-252 |
| 38 | $CH_3$ | O | 2-$CH_3$ | $CH_3$ | $CH_3$ | $CH_3$ | 164-166 |
| 39 | $CH_3$ | O | 3-$CH_3$ | $CH_3$ | $CH_3$ | $CH_3$ | 183-185 |

Tabelle 2 (Fortsetzung)

| Bsp.-Nr. | $R^1$ | X | $R^2_n$ | $R^3$ | $R^4$ | $R^5$ | Schmelzpunkt (°C) |
|---|---|---|---|---|---|---|---|
| 40 | $CH_3$ | S | H | Cl | Cl | Cl | 182-184 |
| 41 | $CH_3$ | S | H | Br | $CH_3$ | $CH_3$ | 160-162 |
| 42 | $CH_3$ | S | H | $SCH_3$ | $CH_3$ | $CH_3$ | |
| 43 | $CH_3$ | S | H | 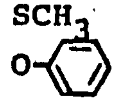 | $CH_3$ | $CH_3$ | 132-134 |
| 44 | $CH_3$ | S | H | $CH_3$ | $CH_3$ | $CH_3$ | 173-175 |
| 45 | $CH_3$ | S | H | $CH_2Cl$ | $CH_3$ | $CH_3$ | 156-158 |
| 46 | $CH_3$ | S | H | $CH_2F$ | $CH_2F$ | $CH_2F$ | 105-107 |
| 47 | $CH_3$ | S | H | $CH_2Cl$ | $CH_2Cl$ | $CH_2Cl$ | 154-156 |
| 48 | $CH_3$ | S | H | 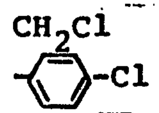 | $CH_3$ | $CH_3$ | 126-128 |
| 49 | $CH_3$ | S | H | 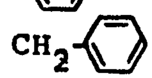 | $CH_3$ | $CH_3$ | 145-146 |
| 50 | $CH_3$ | S | H | $CH_3$ | $-CH_2-CH_2-$ | | 182-184 |
| 51 | $CH_3$ | S | H | $CH_3$ | $-CH_2-CCl_2-$ | | 178-180 |
| 52 | $CH_3$ | S | H | $CH_3$ | $-(CH_2)_4-$ | | 124-126 |
| 53 | $CH_3$ | S | H | $CH_3$ | $-(CH_2)_5-$ | | 130-131 |

Tabelle 2 (Fortsetzung)

| Bsp.-Nr. | $R^1$ | X | $R^2$ | $R^3$ | $R^4$ | $R^5$ | Schmelzpunkt (°C) |
|---|---|---|---|---|---|---|---|
| 54 | $CH_3$ | S | H | $CH_3$ | $-(CH_2)_2-CH(CH(CH_3)_2)-(CH_2)_2-$ | | 172–174 |
| 55 | $CH_3$ | S | 3-Cl | $CH_3$ | $CH_3$ | $CH_3$ | 188–190 |
| 56 | $CH_3$ | S | 2-$CH_3$ | $CH_3$ | $CH_3$ | $CH_3$ | 130–131 |
| 57 | $CH_3$ | S | 2-$CH_3$ | $CH_3$ | $-(CH_2)_4-$ | | 100–102 |
| 58 | $CH_3$ | S | H | $C_3H_7$ | $CH_3$ | $CH_3$ | 100–102 |
| 59 | $CH_3$ | S | H | $C_2H_5$ | $C_2H_5$ | $CH_3$ | 126–128 |
| 60 | H | S | H | $CH_3$ | $CH_3$ | $C_3H_7$ | 86–87 |
| 61 | H | S | H | $CH_3$ | $-(CH_2)_5-$ | | 121–123 |
| 62 | H | S | 3-Cl | $CH_3$ | $CH_3$ | $CH_2Cl$ | 93–94 |
| 63 | H | S | 3-Cl | $CH_3$ | $CH_3$ | $CH_3$ | 124–125 |
| 64 | H | S | 2-$CH_3$ | $CH_3$ | $CH_3$ | $CH_3$ | 92–94 |
| 65 | H | S | 2-$CH_3$ | $CH_3$ | $CH_3$ | $CH_2Cl$ | 122–124 |
| 66 | $CH_3$ | O | H | $CH_3$ | $CH_3$ | 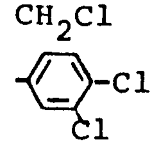 | 146–148 |
| 67 | $CH_3$ | O | H | H | $-(CH_2)_4-$ | | 204–206 |
| 68 | $CH_3$ | S | H | $CH_3$ | F | F | 136–137 |

Tabelle 2 (Fortsetzung)

| Bsp.-Nr. | $R^1$ | X | $R^2$ | $R^3$ | $R^4$ | $R^5$ | Schmelzpunkt (°C) |
|---|---|---|---|---|---|---|---|
| 69 | $CH_3$ | S | H | $CH_3$ | $CH_3$ | 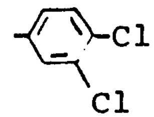 | 152-154 |
| 70 | $CH_3$ | S | H | $CH_3$ | $CH_3$ | 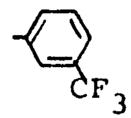 | 144-146 |
| 71 | $CH_3$ | S | H | H | $-(CH_2)_4-$ | | 186-188 |
| 72 | $CF_3$ | S | H | $CH_3$ | $CH_3$ | $CH_3$ | 153-155 |
| 73 | $OCH_3$ | S | H | $CH_3$ | $CH_3$ | $CH_3$ | 184-186 |
| 74 | $SCH_3$ | S | H | $CH_3$ | $CH_3$ | $CH_3$ | 147-149 |

Herstellung von Ausgangsprodukten

Beispiel 75

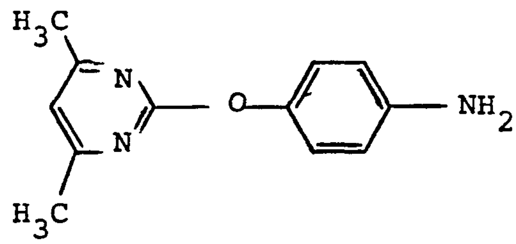

(II-1)

(Verfahren d)

24,5 g (0,1 Mol) 2-(4-Nitro-phenoxy)-4,6-dimethylpyrimidin wurden in 150 ml Dioxan gelöst und mit Wasserstoff in Gegenwart von Raney-Nickel unter einem Druck von 50 bar bei einer Temperatur zwischen 20 und 50°C erschöpfend hydriert. Danach wurde der Katalysator abgesaugt und das Filtrat unter vermindertem Druck eingedampft. Man erhielt 20,4 g (95 % der Theorie) an 2-(4-Amino-phenoxy)-4,6-dimethylpyrimidin in Form einer Festsubstanz.

Schmelzpunkt: 170 - 171°C (nach Umkristallisation aus Essigester)

Nach der im Beispiel 75 angegebenen Methode wurden auch die in den folgenden Beispielen aufgeführten Stoffe der Formel (II) hergestellt.

Beispiel 76

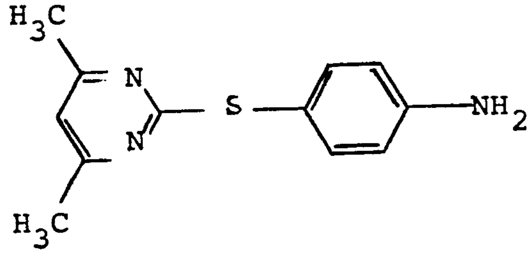

(II-2)

Schmelzpunkt: 170 - 172°C (nach Umkristallisation aus Essigester)

<u>Beispiel 77</u>

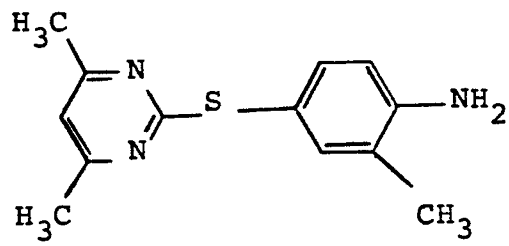

(II-3)

Schmelzpunkt: 134 - 135°C (nach Umkristallisation aus Toluol)

<u>Beispiel 78</u>

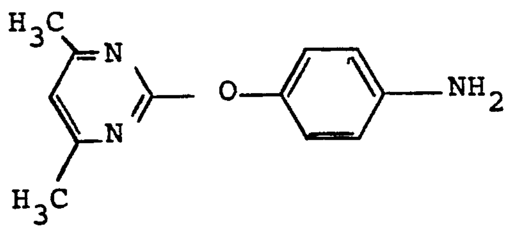

(II-1)

(Verfahren c)

109 g (1 Mol) 4-Amino-phenol wurden in 500 ml Dimethylsulfoxid gelöst. Nach Zugabe von 56 g (1 Mol) pulverisiertem Kaliumhydroxid verrührte man das Gemisch 30 Minuten bei Raumtemperatur und trug dann portionsweise 143 g (1 Mol) 2-Chlor-4,6-dimethyl-pyrimidin ein.

Die Mischung wurde 5 Stunden auf 100°C erwärmt, abgekühlt und dann in 3 Liter Wasser eingerührt. Das Kristallisat wurde abgesaugt, mehrfach mit Wasser gewaschen und getrocknet. Man erhielt 119 g (55,3 % der Theorie) 2-(4-Amino-phenoxy)-4,6-dimethyl-pyrimidin.

Schmelzpunkt: 170 - 171°C (nach Umkristallisation aus Ethanol)

Nach der im Beispiel 78 angegebenen Methode wurden auch die in den folgenden Beispielen aufgeführten Stoffe der Formel (II) hergestellt.

Beispiel 79

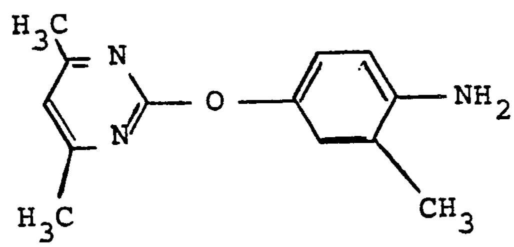

(II-4)

Schmelzpunkt: 226 - 228°C (nach Umkristallisation aus Ethanol)

Beispiel 80

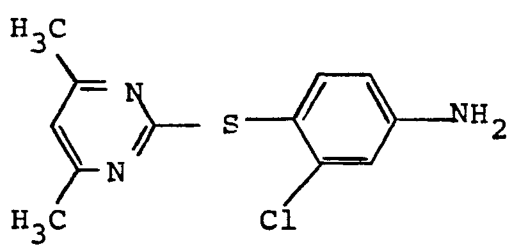

(II-5)

Schmelzpunkt: 167 - 168°C (nach Umkristallisation aus Toluol)

Beispiel 81

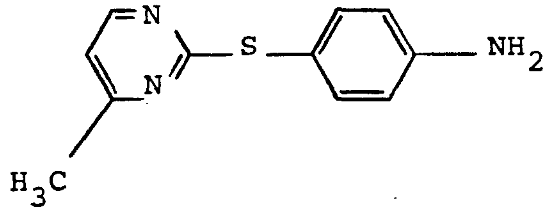

(II-6)

Schmelzpunkt: 73-74°C (nach Umkristallisation aus Tetrachlormethan)

Beispiel 82

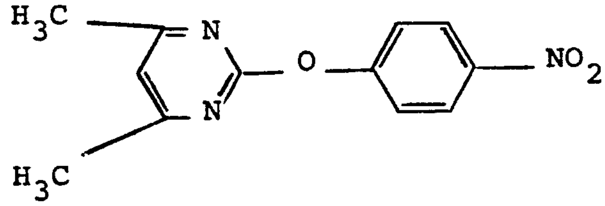

(VIII-1)

27,8 g (0,2 Mol) 4-Nitro-phenol wurden in 100 ml Dimethylsulfoxid gelöst. Nach Zugabe von 11,2 g (0,2 Mol) pulverisiertem Kaliumhydroxid rührte man das Gemisch 30 Minuten bei Raumtemperatur und gab dann 28,5 g (0,2 Mol) 2-Chlor-4,6-dimethyl-pyrimidin hinzu. Die Mischung wurde 6 Stunden auf 120°C erhitzt, dann abgekühlt, mit 1 Liter Wasser verrührt und abgesaugt. Die abgesaugten Kristalle wurden an der Luft getrocknet. Man erhielt 38 g (77,5 % der Theorie) 2-(4-Nitrophenoxy)-4,6-dimethyl-pyrimidin.

Schmelzpunkt: 108 - 110°C.

Beispiel 83

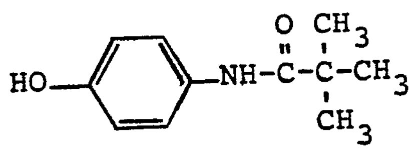

(V-1)

109 g (1 Mol) 4-Amino-phenol und 101 g Triethylamin wurden in 700 ml Tetrahydrofuran gelöst. Hierzu tropfte man bei 10 - 15°C unter Rühren 120,5 g (1 Mol) Pivalsäurechlorid. Die Mischung wurde 2 Stunden bei Raumtemperatur nachgerührt, und dann in 3 Liter Wasser eingegossen. Die abgeschiedenen Kristalle wurden abgesaugt und an der Luft getrocknet. Man erhielt 165 g (85,5 %

der Theorie) 4-Pivaloylamino-phenol.

Schmelzpunkt: 168 - 170°C.

Nach der im Beispiel 83 angegebenen Methode wurden auch die in den folgenden Beispielen aufgeführten Stoffe der Formel (V) hergestellt.

Beispiel 84

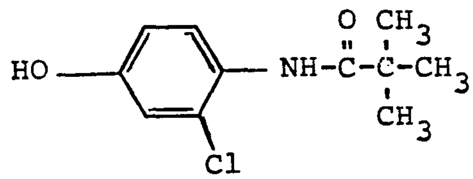

(V-2)

Schmelzpunkt: 122 - 124°C (nach Umkristallisation aus Tetrachlorkohlenstoff)

Beispiel 85

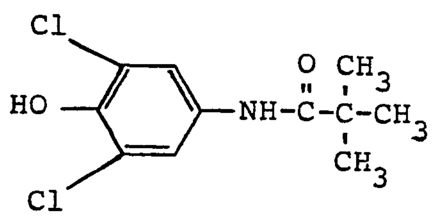

(V-3)

Schmelzpunkt: 182 - 184°C (nach Umkristallisation aus Toluol)

Beispiel 86

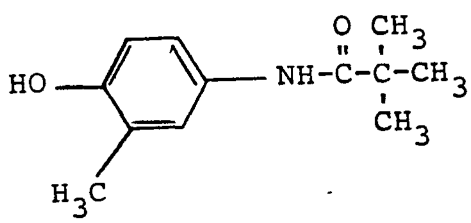

Schmelzpunkt: 150 - 152°C (nach Umkristallisation aus Toluol)

Beispiel 87

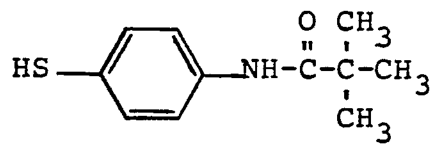

Schmelzpunkt: 127 - 128°C

Verwendungsbeispiel

In dem nachfolgenden Verwendungsbeispiel wurde die folgende Verbindung als Vergleichssubstanz eingesetzt:

(A) =

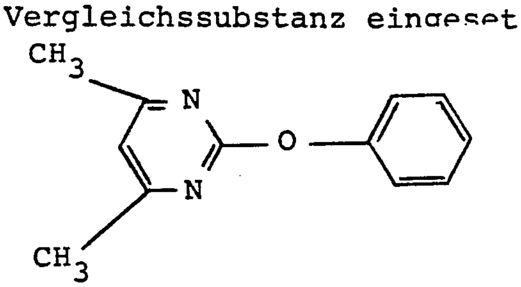

2-Phenoxy-4,6-dimethyl-pyrimidin (bekannt aus US-PS 3, 126, 271)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigt die Verbindung gemäß Beispiel 45 bei der selektiven Bekämpfung von Galinsoga, Galium, Sinapis, Stellaria, Echinochloa, Panicum und Poa in Weizen und Mais eine wesentlich bessere herbizide Wirksamkeit als die Vergleichssubstanz (A).

Beispiel B

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffzubereitung pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der

toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

In diesem Test zeichnen sich die Verbindungen gemäß Beispielen (14), (20) und (38) durch eine sehr gute Wirksamkeit aus.

Patentansprüche

1) Substituierte Carbonsäureanilide der Formel

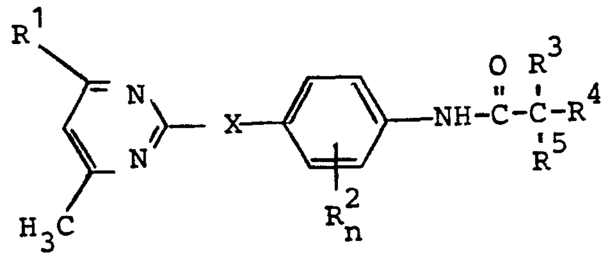

(I)

in welcher

X für Sauerstoff oder Schwefel steht,

$R^1$ für Wasserstoff, Halogen, Alkyl mit 1 bis 6 Kohlenstoffatomen, Trifluormethyl, gegebenenfalls substituiertes Phenyl, Alkoxy mit 1 bis 6 Kohlenstoffatomen oder Alkylmercapto mit 1 bis 6 Kohlenstoffatomen steht,

$R^2$ für Halogen oder Methyl steht,

n für 0, 1 oder 2 steht,

$R^3$ für Wasserstoff, Halogen, gegebenenfalls substituiertes Alkyl mit 1 bis 6 Kohlenstoffatomen, gegebenenfalls substituiertes Aryl, gegebenenfalls substituiertes Aralkyl oder f+r die Reste $-OR^6$ oder $-SO_m-R^6$ steht, wobei

$R^6$ für gegebenenfalls substituiertes Alkyl mit 1 bis 6 Kohlenstoffatomen oder für gegebenenfalls substituiertes Aryl steht und

m für 0, 1 oder 2 steht,

und

$R^4$ und $R^5$ unabhängig voneinander für Halogen oder gegebenenfalls substituiertes Alkyl mit 1 bis 6 Kohlenstoffatomen stehen oder

$R^4$ und $R^5$ gemeinsam mit dem angrenzenden Kohlenstoffatom für einen gegebenenfalls substituierten, gesättigten oder ungesättigten carbocyclischen Ring mit 3 bis 8 Ring-Kohlenstoffatomen stehen.

2) Substituierte Carbonsäureanilide der Formel (I), in denen

X für Sauerstoff oder Schwefel steht,

$R^1$ für Wasserstoff, Fluor, Chlor, Brom, Alkylmit 1 bis 4 Kohlenstoffatomen, Trifluormethyl, gegebenenfalls durch Chlor, Trifluormethyl und/oder Methyl substituiertes Phenyl, Alkoxy mit 1 bis 4 Kohlenstoffatomen oder Alkylmercapto mit 1 bis 4 Kohlenstoffatomen steht,

$R^2$ für Chlor, Brom oder Methyl steht,

n für 0 oder 1 steht,

$R^3$ für Wasserstoff, Fluor, Chlor, Brom, gegebenenfalls durch Fluor, Chlor und/oder Brom substituiertes Alkyl mit 1 bis 4 Kohlenstoffatomen, gegebenenfalls durch Fluor, Chlor, Brom, Trifluormethyl, Methoxy und/oder Methyl substituiertes Phenyl, gegebenenfalls durch Fluor, Chlor, Brom, Trifluormethyl, Methoxy und/oder Methyl substituiertes Benzyl oder für die Reste $-OR^6$ oder $-SO_mR^6$ steht, worin

$R^6$ für gegebenenfalls durch Fluor, Chlor und/oder Brom substituiertes Alkyl mit 1 bis 4 Kohlenstoffatomen oder für gegebenenfalls durch Fluor, Chlor, Brom und/oder Alkyl mit 1 bis 4 Kohlenstoffatomen substituiertes Phenyl steht und

m für 0, 1 oder 2 steht,

und

$R^4$ und $R^5$ unabhängig voneinander für Fluor, Chlor, Brom oder gegebenenfalls durch Fluor, Chlor und/oder Brom substituiertes Alkyl mit 1 bis 4 Kohlenstoffatomen stehen oder

$R^4$ und $R^5$ gemeinsam mit dem angrenzenden Kohlenstoffatom für einen gegebenenfalls durch Fluor, Chlor und/oder Alkyl mit 1 bis 4 Kohlenstoffatomen substituierten gesättigten oder ungesättigten carbocyclischen Ring mit 3 bis 7 Ring-Kohlenstoffatomen stehen.

3) Verfahren zur Herstellung von substituierten Carbonsäureaniliden der Formel

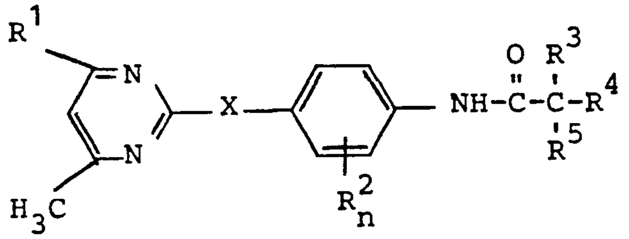

(I)

in welcher

X für Sauerstoff oder Schwefel steht,

$R^1$ für Wasserstoff, Halogen, Alkyl mit 1 bis 6 Kohlenstoffatomen, Trifluormethyl, gegebenenfalls substituiertes Phenyl, Alkoxy mit 1 bis 6 Kohlenstoffatomen oder Alkylmercapto mit 1 bis 6 Kohlenstoffatomen steht,

$R^2$ für Halogen oder Methyl steht,

n für 0, 1 oder 2 steht,

$R^3$ für Wasserstoff, Halogen, gegebenenfalls substituiertes Alkyl mit 1 bis 6 Kohlenstoffatomen, gegebenenfalls substituiertes Aryl, gegebenenfalls substituiertes Aralkyl oder für die Reste $-OR^6$ oder $-SO_m-R^6$ steht, wobei

$R^6$ für gegebenenfalls substituiertes Alkyl mit 1 bis 6 Kohlenstoffatomen oder für gegebenenfalls substituiertes Aryl steht und

m für 0, 1 oder 2 steht,

und

$R^4$ und $R^5$ unabhängig voneinander für Halogen oder gegebenenfalls substituiertes Alkyl mit 1 bis 6 Kohlenstoffatomen stehen oder

$R^4$ und $R^5$ gemeinsam mit dem angrenzenden Kohlenstoffatom für einen gegebenenfalls substituierten, gesättigten oder ungesättigten carbocyclischen Ring mit 3 bis 8 Ring-Kohlenstoffatomen stehen,

dadurch gekennzeichnet, daß man

a) Anilin-Derivate der Formel

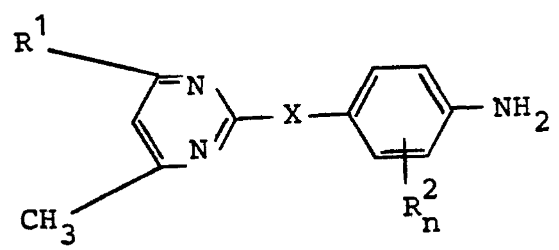

(II)

in welcher

$R^1$, $R^2$, X und n die oben angegebene Bedeutung haben,

entweder

α) mit Säurehalogeniden der Formel

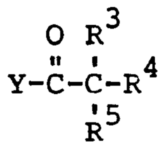 (III a)

in welcher

$R^3$, $R^4$ und $R^5$ die oben angegebene Bedeutung haben und

Y für Halogen steht,

gegebenenfalls in Gegenwart eines Verdünnungsmittels und gegebenenfalls in Gegenwart eines Säurebindemittels umsetzt,

oder

ß) mit symmetrischen Carbonsäureanhydriden der Formel

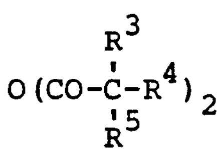 (III b)

in welcher

$R^3$, $R^4$ und $R^5$ die oben angegebene Bedeutung haben,

gegebenenfalls in Gegenwart eines Verdünnungsmittels umsetzt,

γ) mit asymmetrischen Säureanhydriden der Formel

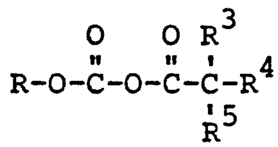

(III c)

in welcher

$R^3$, $R^4$ und $R^5$ die oben angegebene Bedeutung haben und

R für Alkyl mit 1 bis 4 Kohlenstoffatomen oder Phenyl steht,

gegebenenfalls in Gegenwart eines Verdünnungsmittels umsetzt.

oder

δ) mit Verbindungen der Formel

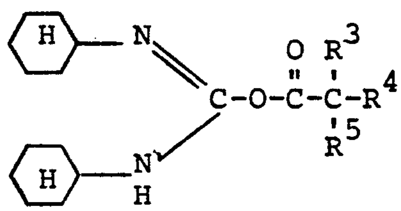

(III d)

in wlcher

$R^3$, $R^4$ und $R^5$ die oben angegebene Bedeutung haben,

gegebenenfalls in Gegenwart eines Verdünnungsmittels umsetzt,

b) Pyrimidin-Derivate der Formel

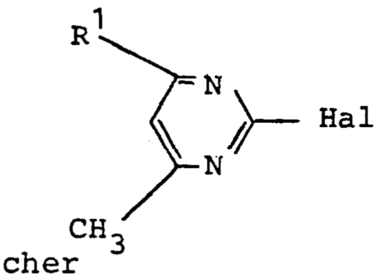

(IV)

in welcher

$R^1$ die oben angegebene Bedeutung hat und

Hal für Halogen steht,

mit Acylanilin-Derivaten der Formel

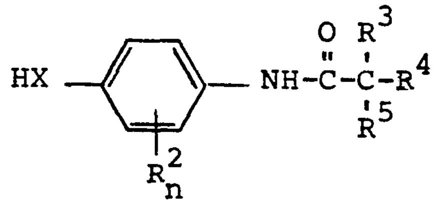

(V)

in welcher

$R^2$, $R^3$, $R^4$, $R^5$, X und n die oben angegebene Bedeutung haben,

in Gegenwart eines Säurebindemittels und gegebenenfalls in Gegenwart eines Verdünnungsmittels umsetzt.

4) Herbizide Mittel, gekennzeichnet durch einen Gehalt an mindestens einem substituierten Carbonsäureanilid der Formel (I) gemäß Anspruch 1 und 3.

5) Verfahren zur Bekämpfung von Unkräutern, dadurch

gekennzeichnet, daß man substituierte Carbonsäureanilide der Formel (I) gemäß Anspruch 1 und 3 auf die Unkräuter und/oder deren Lebensraum ausbringt.

6) Verwendung von substituierten Carbonsäureaniliden der Formel (I) gemäß Anspruch 1 und 3 zur Bekämpfung von Unkräutern.

7) Verfahren zur Herstellung von herbiziden Mitteln, dadurch gekennzeichnet, daß man substituierte Carbonsäureanilide der Formel (I) gemäß Anspruch 1 und 3 mit Streckmitteln und/oder oberflächenaktiven Stoffen vermischt.